# EUROPEAN PATENT SPECIFICATION

(11) **EP 1 495 048 B1**
(45) Date of publication and mention of the grant of the patent: **05.03.2014**
(21) Application number: 03718328.2
(22) Date of filing: 10.04.2003
(51) Int. Cl.: C07K 14/47

(54) **HETEROGENENOUS PROTEIN NETWORKS CROSSLINKED WITH SILICONE-CONTAINING LINKS,AND METHOS FOR PRODUCING THEM**
HETEROGEN DURCH SILIKON VERNETZTE PROTEINE, UND VERFAHREN ZU DEREN HERSTELLUNG
METHODES DE PRODUCTION ET D'UTILISATION DE RESEAUX PROTEIQUES RETICULES HETEROGENES POSSEDANT DES GROUPES DE LIAISON CONTENANT DU SILICONE

(30) Priority: 10.04.2002 US 119477; 22.04.2002 US 127523; 26.04.2002 US 133885; 05.07.2002 US 393958 P
(43) Date of publication of application: 12.01.2005
(73) Proprietor: Keraplast Technologies Ltd., San Antonio, TX 78258 (US)
(72) Inventor: VAN DYKE, Mark, E., Fair Oaks Ranch, TX 78015 (US)
(74) Representative: Glawe, Delfs, Moll
(86) International application number: PCT/US2003/011102
(87) International publication number: WO 2003/087195

(56) References cited:
- EP-A- 0 298 684
- EP-A- 0 312 962
- EP-A- 0 540 357
- WO-A-01/19283
- US-A- 3 677 693
- US-A- 4 032 676
- US-A- 5 300 285
- US-A1- 2002 028 243
- US-A1- 2003 204 037
- US-B1- 6 352 699
- MENEFEE, E. AND YEE, G.: "Cross - linking in keratins. IV. Thermal cleavage of cross - links" JOURNAL OF APPLIED POLYMER SCIENCE, vol. 9, no. 8, 1965, pages 2847-2853, XP009018496
- ALFERIEV I S ET AL: "High reactivity of alkyl sulfides towards epoxides under conditions of collagen fixation-a convenient approach to 2-amino-4-butyrolactones" BIOMATERIALS, ELSEVIER SCIENCE PUBLISHERS BV., BARKING, GB, vol. 22, no. 18, September 2001 (2001-09), pages 2501-2506, XP004273577 ISSN: 0142-9612

## Description

### Cross Reference to Related Applications

The present application claims the priority date of the following application: U.S. Patent Application Serial No. 10/133,885, filed April 26, 2002. Related applications, to which priority is claimed as maybe required, are U.S. Patent Application Serial No. 10/127,523, filed April 22,2002, U.S. Patent Application Serial No. 10/119,477, filed April 10, 2002; and U.S. Provisional Application No. 60/393,958, filed July 5, 2002. A related applications is Application Serial No. 10/254,364, filed September 25,2002, claiming the benefit of U.S. Provisional Application No. 60/324,709.

### Field of the Invention

The invention is set out in the appended claims. The embodiments of the description which do not fall within the scope of the claims are provided for illustrative purposes only and do not form part of the present invention.

The present description is directed to methods for producing biocompatible heterogeneous proteinaceous networks crosslinked with a heterogeneous crosslinking agent other than glutaraldehyde, preferably a silicone-based crosslinking agent. Preferred proteins for use in forming the networks are α-keratins, or high molecular weight keratins (HMWK's). The crosslinking agent preferably reacts with reactive pendant groups existing on the keratin molecules and either produces no byproducts, produces biocompatible byproducts, such as hydrogen, water, and carbon dioxide, or produces byproducts that can be removed from the network.

### Background of the Invention

Proteins, such as keratin proteins, are beneficial in healing damaged epithelial tissues. Unfortunately, the chemical and engineering properties of keratin proteins have been relatively limited to those achieved using oxidative and reductive chemistries, and side chain protein crosslinks. A need exists for proteins, and methods for crosslinking proteins, preferably α-keratins, to form films having a broad scope of chemical and engineering properties so that the potential applications of protein-based materials can be expanded.

EP-A-0 540 357 and US-A-5 300 285 disclose the use of protein-silicone copolymers in a person's hair care. The purpose,of the hair coating in EP-A-0 540 357 is to add shine and texture to hair and US-A-5 300 285 describes a way to reform disulfide bridges on a person's hair with less harsh conditions. None of the documents EP-A-0 540 357 and US-A-5 300 285, neither alone nor in combination with each other discloses or suggests the production and intended use of the proteinaceous film as defined in the claims, in particular "wound healing" which is completely different from the use in EP-A-0 540 357 and US-A-5 300 285.

The invention is set out in the appended claims. The embodiments of the description which do not fall within the scope of the claims are provided for illustrative purposes only and do not form part of the present invention.

### Summary of the Invention

A method is provided for making a keratin network interlinked by a silicone-based crosslinking agent, said method comprising exposing a plurality of α-keratin molecules comprising reactive pendant groups to a multi-functional silicone-based crosslinking agent under conditions effective to form covalent interprotein crosslinks between first reactive functionalities on said crosslinking agent and first reactive pendant groups on a first group of said α-keratin molecules, said conditions also being effective to form covalent interprotein crosslinks between second reactive functionalities on said crosslinking agent and second reactive pendant groups on a second group of α-keratin molecules.

Also provided are networks comprising proteinaceous material consisting essentially of α-keratin molecules comprising interprotein crosslinks comprising first covalent bonds between first reactive functionalities on a plurality of molecules of a silicone-based crosslinking agent and first reactive pendant groups on a plurality of first α-keratin molecules and second covalent bonds between second reactive functionalities on a plurality of molecules of said silicone-based crosslinking agent and second reactive pendant groups on a plurality of second α-keratin molecules.

### Detaited Description of the Intention

The invention is set out in the appended claims. The embodiments of the description which do not fall within the scope of the claims are provided for illustrative purposes only and do not form part of the present invention.

The present description is directed toward methods for crosslinking proteins, preferably using heterogeneous crosslinking agents other than glutaraldehyde to form heterogeneous proteinaceous networks or films. As used herein, the term "heterogeneous" refers to a proteinaceous network or film, preferably comprising protein molecules having a relatively high molecular weight of at least about 50 kDa, preferably from about 50 to about 85 kDa, or derivatives therefrom. The protein molecules are interlinked by a silicone-based crosslinking material.

The methods described herein may be used to treat a wide variety of proteins to form network structures, preferably elastomeric films. Examples of suitable naturally occurring proteins include, but are not necessarily limited to keratin, collagen, and elastin. The proteins may be natural, synthetic, or recombinant. Preferred proteins are relatively high in cysteine content. Most preferred proteins are keratin proteins, even more preferably α-keratin proteins, also sometimes called high molecular weight keratins (HMWK's).

A preferred source of keratin proteins is hair or fur. The hair may be animal, or human. Keratins are loosely defined as the hardened and insolubilized proteins found in the epidermal cells of vertebrates. Human hair is composed almost entirely of keratins.

Human hair has a cuticle, which is a tough tubular outer layer made up of flattened cells arranged in a scaly, overlapping profile. The inner bulk of the hair is called the cortex and is constructed from elongated cells that are densely packed with fibrous keratins. The fibrous keratins are arranged in bundles referred to as microfibrils and possess an α-helical tertiary structure. The microfibrils are bound together with an amorphous keratin matrix.

The amorphous keratin matrix and the microfibrils vary in function and composition. The matrix is the "glue" that holds the microfibrils together. This matrix "glue" is high in sulfur content, and is comprised of low molecular weight keratins (LMWK) which typically have an average molecular weight of from about 10 to about 15 kDa. The microfibrils are comprised of high molecular weight keratins (HMWK) having a relatively lower sulfur content, but having a higher average molecular weight of typically from about 50 to about 85 kDa. HMWK's and LMWK's vary in chemical properties, such as reactivity and solubility.

Keratins are afforded their structural integrity, in large part, by the presence of disulfide crosslinks which form a three dimensional network of polypeptide chains. This network structure renders keratins insoluble. Keratins can, however, be made water soluble by destroying this three dimensional structure via disulfide bond scission. Disulfide bond scission can be performed either oxidatively, reductively, or using some combination of both types of bond scission. Oxidative bond scission with hydrogen peroxide, for example, results in the formation of sulfonic acid residues produced from cystine. The material produced using hydrogen peroxide for disulfide bond scission is highly ionic and has excellent water solubility. Reductive bond scission with mercaptoethanol, for example, results in the formation of cysteine residues produced from cystine. The material produced using this reductive technique is highly reactive and will readily re-crosslink.

### Disulfide bond scission and keratin extraction

The proteins, preferably α-keratins, may be processed and/or isolated in any manner that renders them sufficiently soluble in the reaction media for crosslinking reaction(s) to occur. A number of the reactions described below call for an anhydrous solvent. Persons of ordinary skill in the art will recognize that anhydrous solvents include a large number of solvents, including, but not necessarily limited to 1,2,-dimethoxyethane, dimethylformamide, dimethylsulfoxide (DMSO), N-methyl pyrrolidone, and others. generally, the reactions require the presence of at least some water.

### Oxidation/Reduction of Cystine Residues

Keratins can be used as a source material (e.g. human hair), and the hair may be oxidized by a suitable oxidizing agent. Suitable oxidizing agents include, but are not necessarily limited to hydrogen peroxide, peracetic acid, percarbonates, persulfates, chlorine dioxide, sodium and calcium peroxides, perborates, and hypochlorite. The oxidants are used at concentration of up to about 35%, preferably at from about 0.1% to about 10%. The oxidation preferably occurs at reflux temperatures.

In a preferred embodiment, the hair is treated with hydrogen peroxide (H₂O₂), at from about 0.1% to about 10%, most preferably 1%, in order to disrupt the cuticle and swell the keratin source material. This process also converts some fraction of the cystine residues into sulfonic acid groups. The amount of oxidation may be controlled by varying the time of oxidation, preferably from about 0 hours to about 4 hours, while retaining the other conditions of the oxidation reaction constant. These conditions include concentration and type of oxidant, temperature, and ratio of extracting media to keratin source material. After the reaction is complete, the oxidized hair is filtered and rinsed, preferably with deionized water. The filtrate is discarded and the hair allowed to dry.

Where other conditions of oxidation are maintained constant, the conversion rate of cystine to sulfonic acid residues is roughly proportional to the amount of time used for the oxidation. Residual cystines in the resulting oxidized keratin solids are converted to other sulfur-containing moieties using reductive techniques. Preferably, the disulfide-bridged cystine group is converted to a thiol group, which has utility of it's own, or can be modified using a variety of chemical techniques.

### Reaction with a Reducing Agent

If oxidized, the oxidized hair preferably is treated with a reducing agent. Treatment of oxidized keratin proteins with reducing agents facilitates the formation of cysteine from cystine, but tends to leave the previously oxidized groups unaltered. Suitable reducing agents include, but are not necessarily limited to thioglycolic acid and salts thereof, mercaptoethanol, dithiothreitol, thioglycerol, thiolactic acid, glutathione, cysteine, sodium sulfide, and sodium hydrosulfide. Preferred reducing agents are thioglycolic acid and mercaptoethanol, most preferably thioglycolic acid.

In order to treat the oxidized hair with the reducing agent, the previously oxidized hair is suspended in the reducing agent typically at a concentration of up to about 10N, preferably from about 0.1N and 1N; at a pH greater than about 7, preferably equal to or greater than 9, most preferably 9; a temperature of from about 25 to about 80 °C, preferably about 60 °C, preferably for a time period of from about 1 to about 72, most preferably about 24 hours. The reaction occurs under an inert atmosphere, preferably nitrogen. The liquid fraction is separated from any remaining solids using known means, including but not necessarily limited to filtration, or cannulation and/or centrifugation, preferably under inert atmosphere. A preferred method of separation is filtration. Once the solids are removed, the soluble keratin proteins are isolated from the solution by addition of a water-miscible non-solvent, or by spray drying. Water-miscible non-solvents include, but are not necessarily limited to ethanol, methanol, isopropyl alcohol, tetrahydrofuran, acetone, dioxane, and the like, again under inert atmosphere. A preferred non-solvent is ethanol. The precipitate is separated from the non-solvent using known means, preferably by filtration and rinsing using additional aliquots of the non-solvent. The resulting keratin proteins are dried using known techniques, preferably overnight under vacuum at room temperature. This process results in the keratins having both sulfonic acid groups and thiol groups.

Thiols possess reactivities similar to alcohols, and can be used to perform a multitude of known organic chemical reactions, such as those described in McMurry, J., Organic Chemistry, Brooks/Cole Publishing Co., Monterey, CA (1984); Scudder, P. H., Electron Flow in Organic Chemistry, John Wiley & Sons, New York, NY (1992); Stowell, J. C., Intermediate Organic Chemistry, John Wiley & Sons, New York, NY (1994). The ratio of sulfonic acid to thiol is primarily controlled by the quantity of primary reactive sites remaining after oxidation. Of course, the rate of reduction will also be affected by reagent concentration(s), reaction temperature(s), and exposure time(s).

### Reductive/reductive extraction

Reductive chemistries also are known for disulfide bond scission in keratins: See Wardell, J. L., "Preparation of Thiols" in The Chemistry of the Thiol Group, Patai, S. (Editor), pp. 163-353, John Wiley & Sons, New York, NY (1974). HMWK's may be extracted from hair using at least two reductive extractions, as described in Crewther, W. G., Fraser, R. D. B., Lennox, F. G., and Lindley, H., "The Chemistry of Keratins" in Advances in Protein Chemistry, Anfinsen, C. B., Jr., Anson, M. L., Edsall, J. T., and Richards, F. M. (Editors), Academic Press, New York, pp. 191-346 (1965).

Suitable reducing agents include, but are not necessarily limited to thioglycolic acid and salts thereof, mercaptoethanol, dithiothreitol, thioglycerol, thiolactic acid, glutathione, cysteine, sodium sulfide, and sodium hydrosulfide, Preferred reducing agents are thioglycolic acid and mercaptoethanol, most preferably thioglycolic acid.

In order to selectively reduce and extract the desired proteins, the hair (or other protein source) is suspended in a reducing agent at a concentration of from about 0.1N to about 10N, preferably about 1.0N. Gentle swelling of hair fibers is achieved at a pH of about 9 or more, preferably at a pH of from about 9 to about 10.5. Hence, the initial reduction takes place at a temperature of from about 20 to about 100 °C, preferably at about 25 °C. The time period required to accomplish the first reduction is from about 4 to about 24 hours, most preferably about 12 hours. The reaction occurs under an inert atmosphere, preferably nitrogen. The liquid fraction is separated from remaining solids using known means, including but not necessarily limited to filtration, cannulation, and/or centrifugation, preferably under inert atmosphere. A preferred method of separation is filtration.

A second extraction is performed on the reduced solids using a suitable swelling agent, preferably urea, bases such as ammonium hydroxide, sodium hydroxide, or potassium hydroxide. A most preferred swelling agent for this second extraction is concentrated urea. The second extraction effectively removes the reduced fibrous α-keratins from inside the cuticle. The second extraction occurs at from about 1M to about 10M urea, preferably about 7M urea, for a period of at least about 1 hour, preferably from about 1 to about 72 hours, most preferably about 24 hours. The second extraction occurs at room temperature, but may take place at temperatures of from about 20 °C to about 100 °C, preferably about 25 °C. The liquid fraction is separated from the empty, intact cuticle, using known means. Suitable means include but are not necessarily limited to filtration, cannulation and/or centrifugation, preferably under inert atmosphere. A preferred method of separation is filtration.

Once the cuticle is removed, the extracted keratin proteins may be retained in solution for further use, or they may be isolated from the solution by addition to a water-miscible non-solvent, or by spray drying. Water-miscible non-solvents include, but are not necessarily limited to ethanol, methanol, isopropyl alcohol, tetrahydrofuran, acetone, dioxane, and the like, again under inert atmosphere. A preferred non-solvent is ethanol. The precipitate is separated from the non-solvent using known means, preferably by filtration and rinsing using additional aliquots of the non-solvent. The precipitated proteins are dried using known techniques, preferably overnight under vacuum at room temperature. The dried keratin proteins are ground into a powder, sometimes referred to as "HMWK powder."

### Silicone-Based Crosslinking Agents

In a most preferred embodiment, the crosslinking agent is a multifunctional silicone-based material. Silicones are a family of biocompatible materials that have been used in a myriad of medical applications. Silicone gel sheeting, a form of lightly crosslinked silicone polymer, promotes wound healing and lessens the degree of hypertrophic scar formation. The technology of silicone chemistry is varied and useful, particularly with respect to elastomer formation, as many crosslinking modalities have been developed. Thomas, D.R., "Cross-linking of Polydimethylsiloxanes"; in Siloxane Polymers, Clarson, S. J. and Semlyen, J. A. (Editors), PTR Prentice Hall, New Jersey, pp. 567-615(1993). Many of these crosslinking chemistries can be adapted for use in other systems such that copolymer and interpenetrating networks comprising at least some silicone have been produced. The beneficial wound healing attributes of silicone biomaterials, combined with their flexible chemistry, makes them ideal candidates for crosslinking keratin-based biomaterials.

Silicones are bioinert and resilient in biological systems. A bioinert crosslinking agent has the advantage of maintaining the biological stealth of the system of which it is a part. The combination of keratins with silicone-based crosslinking agents combines the wound healing efficacy of both biomaterials without compromising the inherent biocompatibility of keratins.

Suitable silicone cross-linking agents, or polysiloxanes, are molecules having recurring Si-O linkages: 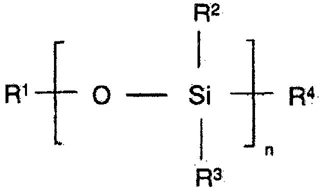 wherein n is from about 1 to about 50, and R¹, R², R³, and R⁴ can be a large variety of groups, wherein at least two of R¹, R², R³, and R⁴ comprise a "reactive functionality," defined as a functionality that is reactive toward reactive pendant groups on the protein molecules to be interlinked. Suitable reactive functionalities comprise one or more reactive moieties selected from the group consisting of reactive unsaturated carbon-carbon bonds, reactive oxygens, reactive nitrogens, reactive sulfurs, and reactive halogens. Preferred reactive functionalities include, but are not necessarily limited to reactive unsaturated carbon-carbon bonds, hydrido groups, hydroxyl groups, alkylamine groups, alkylmercapto groups, alkoxy groups, trifluoroalkyl groups, wherein the alkyl moiety comprises from about 1 to about 6 carbon atoms. A preferred trifluoroalkyl group is a trifluoropropyl group; a preferred alkoxy group is an epoxy group, and a preferred unsaturated carbon-carbon bond is a vinyl group.

Examples of suitable R¹, R², R³, and R⁴ groups, some of which are reactive functionalities and some of which are not, include, but are not necessarily limited to hydrogen; cyclic, linear, and branched alkyl and heteroalkyl groups having from about 1 to about 6 carbon atoms, said groups comprising both unsubstituted groups and groups substituted with at least one reactive functionality, wherein said heteroalkyl groups comprise one or more heteroatoms selected from the group consisting of nitrogen, oxygen, and sulfur; cyclic, linear, and branched alkenyl and heteroalkenyl groups having from about 2 to about 6 carbon atoms, and mercapto functionalized versions thereof and resonance hybrids thereof, said groups comprising both unsubstituted groups and groups substituted with at least one reactive functionality; carboxyl groups and salts, esters, and amides thereof comprising cyclic, linear, and branched alkyl groups, heteroalkyl groups, alkenyl groups, and heteroalkenyl groups having from about 1 to about 6 carbon atoms wherein said hetero groups comprise one or more heteroatoms selected from the group consisting of nitrogen, oxygen, and sulfur; aromatic groups; alkanols and alkenols having from about 1 to about 6 carbon atoms; alkanolamides and alkenol amides having from about 1 to about 6 carbon atoms; and combinations thereof; alkoxy groups (sometimes referred to herein as "alkyl ethers") comprising one or more alkyl moieties having a total of from about 1 to about 6 carbon atoms, hydrido groups, and hydroxyl groups. Preferred heteroalkyl groups include, but are not necessarily limited to acetoxy groups, silane groups optionally comprising one or more alkyl substitutent having a total of from about 1 to about 6 carbon atoms, and combinations thereof. Preferred alkoxy groups include, but are not necessarily limited to epoxy groups.

Preferably, R¹ and R⁴ are moieties comprising reactive functionalities which are adapted to react with complementary functional groups on the protein molecules to be interlinked, preferably α-keratin molecules. In a preferred embodiment, R¹ and R⁴ independently are selected from the group consisting of hydrogen, linear, branched or cyclic alkyl groups having from about 1 to about 6 carbon atoms, alkenyl groups having from about 2 to about 6 carbon atoms, hydrido groups, alkoxy groups comprising one or more alkyl groups having a total of from about 1 to about 6 carbon atoms, hydroxy groups, alkylamine groups, alkylmercapto groups, acrylate groups, methacrylate groups, halo groups, acetoxy groups, and epoxy groups. In a more preferred embodiment, both R¹ and R⁴ comprise a moiety selected from the group consisting of vinyl groups and epoxy groups. Most preferably, R¹ and R⁴ comprise the same moiety selected from the group consisting of vinyl groups and epoxy groups; and

In a preferred embodiment, R² and R³ independently are selected from the group consisting of hydrogen, cycloalkyl groups, vinyl groups, hydrido groups, trifluoroalkyl groups, phenyl groups, alkyl groups, alkoxy groups, alkylmercapto groups, and alkylamine groups; provided that, when one of R² or R³ is a vinyl group, the other of R² or R³ is a group other than a hydrido group; and, when one of R² or R³ is a hydrido group, the other of R² or R³ is a group other than a vinyl group. In an even more preferred embodiment, R² and R³ preferably are relatively inert groups. Most preferably at least one of R² and R³ is an alkyl group, more preferably a methyl group.

Commercially available silicone products include, but are not necessarily limited to vinyl functional, alkoxy functional (preferably epoxy functional), alkylamine functional, hydroxyl functional, and alkylmercapto functional polysiloxy polymers and copolymers, which are available, for example, from Gelest, Inc., Tullytown, PA, or may be made using known methods, such as those described in Thomas, D. R., "Cross-linking of Polydimethylsiloxanes", in Siloxane Polymers, Clarson, S. J. and Semlyen, J. A. (Editors), PTR Prentice Hall, New Jersey, pp. 567-615 (1993). Most preferred commercially available vinyl functional products are generally available in molecular weights ranging from about 363 to about 5,500, with preferred molecular weights being from about 500 to about 3500.

A preferred crosslinking agent is the epoxycyclohexyl copolymer discussed in more detail below. More preferred is epoxypropoxypropyl-terminated silicones discussed in more detail below. Most preferred crosslinking agents are vinyl-terminated silicones. These crosslinking agents may be obtained, for example from Gelest, Inc., Tullytown, Pa., or prepared using known procedures.

(Epoxycyclohexylethyl)methylsiloxane-dimethylsiloxane copolymer generally is available in molecular weights ranging from 500 to 50,000, with preferred molecular weights being from about 650 to about 3500. (Epoxycyclohexylethyl)methylsiloxane-dimethyl siloxane copolymer has the following general structure: 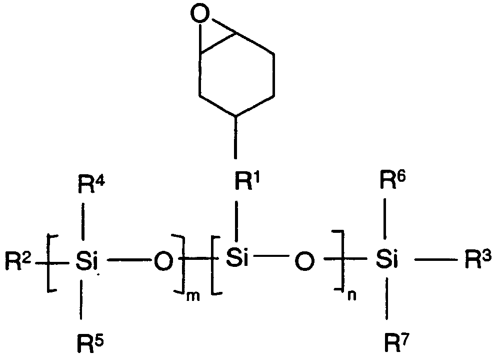 wherein m and n add to a total of from about 5 to about 50; R² and R³ may be any of the groups listed as end groups R¹ and R⁴ in the general formula for the silicone cross-linking agents given at the beginning of this section, and R¹, and R⁴-R⁷ may be any of the substitutents listed as the Si-substitutents R² and R³ in the general formula for the silicone cross-linking agents given at the beginning of this section. Preferably, R¹ and R⁴-R⁷ are selected from the group consisting of alkyl groups having from about 1 to about 6 carbon atoms. R¹ preferably is a methyl group; R⁴-R⁷ preferably are methyl groups. A preferred (epoxycyclohexylethyl)methylsiloxane-dimethylsiloxane copolymer, which is commercially available from Gelest, has the following general structure:

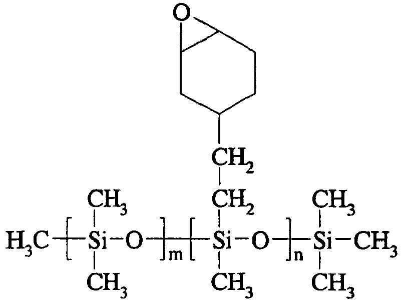

Preferred silicone-based crosslinking agents react with reactive groups on the protein molecules to produce biocompatible byproducts, preferably hydrogen, water, carbon dioxide, and/or any other biocompatible byproduct that is readily metabolized or excreted, removed from the network, or at least is not toxic to the human body. Suitable silicone-based crosslinking agents have either two or more of the same reactive functionalities, or two or more different reactive functionalities. Preferred silicone-based crosslinking agents have two or more of the same functional group.

### Network formation

Thiols and other chemical moieties contained in amino acid residues have utility as labile sites for crosslinking reactions to form protein networks, preferably networks having the properties of an elastomeric film. Preferred networks are made using HMWK proteins.

Once the desired crosslinking agent(s) are determined, proteins, preferably HMWK proteins, are dissolved in a suitable solvent. For most reactions, a preferred solvent is an aqueous solvent. In the case of silicone-based crosslinking agents, a preferred solvent is an anhydrous solvent comprising a base. Preferably, about 2 g of HMWK powder is mixed in the solvent containing a suitable base, and the mixture is stirred and heated to a temperature effective to dissolve the keratin, typically not more than 60°C. The pH of the solution is maintained at about 9 to 11 using a suitable base. Suitable bases include, but are not necessarily limited to ammonium hydroxide, sodium hydroxide, and potassium hydroxide, preferably ammonium hydroxide. At least about 5 wt.%, preferably about 10 wt.%, relative to the keratin, of a multifunctional crosslinking agent is added to the mixture, forming a network precursor solution. Depending upon the crosslinking agent, a catalyst or promoter may be added. The network precursor solution is distributed over an appropriate surface or mold, preferably to a thickness of from about 1 to about 10 mm, and cured by exposure to suitable energy, such as a heat lamp, an autoclave, a microwave, or a UV lamp. In a preferred embodiment for making films comprising a silicone-based crosslinking agent, the solutions are irradiated for a period of from about 1 hours to about 8 hours, preferably about 2 hours under a UV lamp (λ = 365 nm) and then dried under a heat lamp effective to produce a temperature of at least about 60°C for a period of from about 30 minutes to about 300 minutes, preferably about 4 hours.

Alternately, the keratin is dissolved in water and the silicone is dissolved in a separate solution of water-miscible organic solvent. Suitable organic solvents include ethanol, methanol, isopropyl alcohol, acetone, tetrahydrofuran, and dimethylsulfoxide. The two solutions are then mixed and a film cast from the resulting mixture.

### Crosslinking reactions

Crosslinking of the proteins and network formation occurs, generally, when a silicone-based crosslinking agent which is at least difunctional, or has at least two reactive groups, is used to crosslink between reactive pendant groups on two different keratin molecules. The silicone based reactant creates a bridge between keratin molecules, or an interprotein cross-link, and thus produces a three-dimensional network.

Proteins comprise amino acids, which generally have the formula:

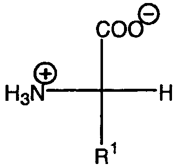

Table 1 summarizes the amino acid residues found in human hair, for example, and shows the "R¹" groups associated with each residue.

| Table 1. Ranked average amounts of amino acids in human hair | | | | | |
|---|---|---|---|---|---|
| Amino Acid | R¹ Group | Nature | pKa | Iso-electric Point (pH) | Percent Composition in Hair |
| Cysteine | H-S-CH₂- | Nonpolar | 8.4 | 5.02 | 17.3 |
| Glutamic Acid | 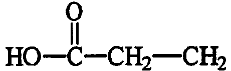 | Polar | 4.5 | 3.22 | 13.9 |
| Arginine | 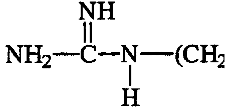 | Polar | 12.5 | 11.15 | 9.85 |
| Serine | HO-CH₂- | Polar | None | 5.68 | 9 |
| Threonine | 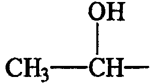 | Polar | None | 5.64 | 7.75 |
| Leucine | 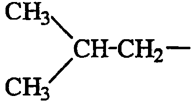 | Hydrophobic | None | 5.98 | 7.35 |
| Proline | 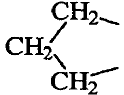 | Hydrophobic | None | 6.3 | 6.95 |
| Aspartic Acid | 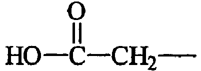 | Polar | 4.5 | 2.77 | 5.8 |
| Valine | 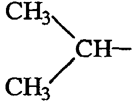 | Hydrophobic | None | 5.96 | 5.7 |
| Isoleucine | 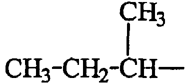 | Hydrophobic | None | 5.94 | 4.75 |
| Glycine | H- | Nonpolar | None | 5.65 | 4.15 |
| Phenylalanine | 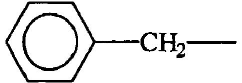 | Hydrophobic | None | 5.48 | 3 |
| Alanine | CH₃- | Hydrophobic | None | 6 | 2.8 |
| Tyrosine | 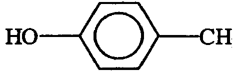 | Hydrophobic | None | 5.66 | 2.6 |
| Lysine | NH₂-(CH₂)₄- | Polar | 10.4 | 9.59 | 2.5 |
| Histidine | 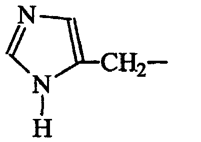 | Aromatic | 6.2 | 7.47 | 0.9 |
| Methionine | CH₃-S-CH₂-CH | Hydrophobic | None | 5.74 | 0.85 |
| Tryptophan | 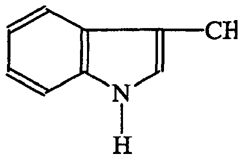 | Hydrophobic | None | 5.89 | 0.85 |

The most abundant amino acid in human hair is cysteine, which is found in the form of disulfide-bridged cystine groups. As discussed above, this group can be converted to other sulfur containing moieties, most notably thiol. Thiols theoretically can be reacted with reactive ends of a crosslinking agent using a number of chemical techniques, such as those described in S. Patai (Ed.), the Chemistry of the Thiol Group, Parts 1 and 2, John Wiley & Sons, New York, NY (1974). Other reaction scenarios, such as those directed toward polymer synthesis, also are useful to convert thiol groups and other pendant groups to an assortment of desirable functional residues, including those described in Rempp, P. and Merrill, E. W., Polymer Synthesis, Huethig & Wepf Verlag Basel, Heidelberg, Germany (1986); Young, R. J. and Lovell, P. A., Introduction to Polymers, Chapman & Hall, London (1991); Odian, G., Principles of Polymerization, John Wiley & Sons, New York, NY (1991).

In addition to cysteine, the following amino acids have pendant groups comprising nitrogen or oxygen which may be useful as reactive pendant groups; arginine, serine, glutamic acid, threonine, aspartic acid, lysine, asparagine, glutamine, tyrosine, tryptophan, and histidine. Where the protein is α-keratin, preferred amino acid residues comprising reactive pendant groups for crosslinking are cysteine, arginine, serine, and glutamic acid, most preferably cysteine and arginine.

The silicone-based crosslinking agents comprise at least two reactive functionalities. For convenience, the crosslinking agents described herein sometimes are referred to as "di-" functional. However, unless a crosslinking agent is expressly claimed or expressly stated to be di-functional only, it is to be understood that the crosslinking agents described herein may also be multi-functional, e.g., di-, tri, tetra-, etc.

Without limiting the invention to a particular theory or mechanism of action, unless expressly claimed, the following are crosslinking chemistries involved in producing the heterogeneous crosslinked protein networks:

### Production of thioether

A preferred reductive modification is the formation of a thiolate anion, followed by nucleophilic substitution employing an appropriate leaving group, yielding a thioether, preferably an alkoxy functional thioether (or a thioester). A preferred alkoxy functional thioether is an epoxy-functional thioether. The general reaction is shown below: 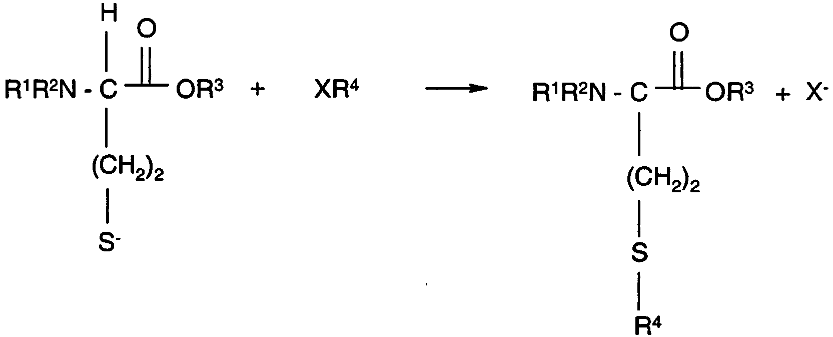 wherein R¹ and R² comprise entities selected from the group consisting of hydrogen and the remainder of the N-terminal portion of the protein molecule; R³ comprises the remainder of the carboxy-terminal portion of the protein molecule; and, R⁴ is a group adapted to form a thioether, preferably an alkoxy functional thioether. Suitable R⁴ groups comprise a "substitution end," which bonds with the sulfur and a "reactive end" which reacts with the crosslinking agent. Suitable substitution ends include, but are not necessarily limited to unsubstituted and halo-substituted alkyl groups and alkylene groups having from about 1 to about 8 carbon atoms, including resonance hybrids, such as allyl groups, and unsubstituted and halo-substituted aryl groups. Suitable reactive ends include, but are not necessarily limited to acyl groups, and polyalkylethers containing from about 1 to 50 repeat groups, silane groups, and silicone groups. Preferred reactive ends include, but are not necessarily limited to carboxyl groups, hydroxyl groups, and alkoxide groups. A most preferred reactive end is an epoxide group. In the foregoing formula, X may be any appropriate leaving group. Suitable leaving groups include, but are not necessarily limited to halide groups, tosylate groups, acetate groups, hydroxyl groups, alkoxy groups, and amine groups. Preferred X groups are halides, most preferably chlorine. In a most preferred embodiment, XR⁴ is epichlorohydrin.

The thiolate anion can be generated from thiol, or more directly from the water soluble protein feedstock, preferably a keratin feedstock, by reaction with a reactive nucleophile. Suitable nucleophiles include alkyl and aryl functional sulfide salts, sulfonates, isocyanates, thiocyanates, halides, hydrosulfide, hydroxide, alkoxides, azides, and acetates preferably alkyl and aryl sulfide salts, hydrosulfide, hydroxide, alkoxides, azides, and acetates. A most preferred nucleophile is sodium sulfide.

The reaction where RX is epichlorohydrin is shown below: 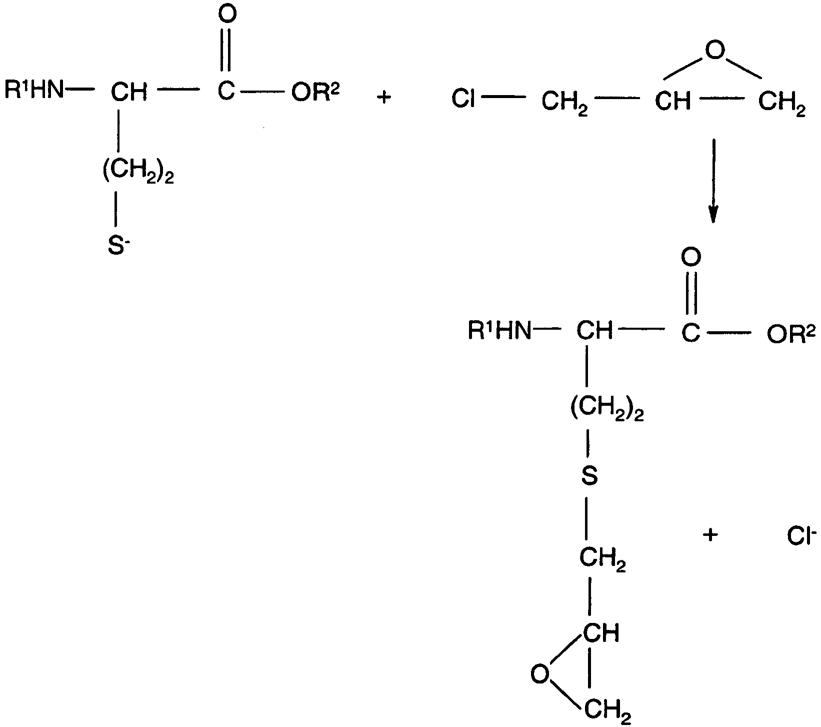 wherein R¹ and R² are the remainder of the water soluble protein molecule of which cysteine is a part.

In order to form the foregoing epoxide functionalized water soluble proteins, preferably water soluble keratins, a water soluble keratin source material is first produced, preferably as described above. The water soluble keratins are then exposed to a solution of "RX", preferably epichlorohydrin, in aqueous solution at a pH of from about 9 to about 11. The RX is typically at a concentration of up to about 20 mole percent relative to keratin, preferably from about 5 to 10 mole percent relative to keratin, most preferably about 10 mole.%. The pH is greater than about 7, preferably greater than about 9. The temperature is from about 20 to about 100 °C, preferably about 60°C. The reaction continues for a time period of from about 1 to about 72 hours, most preferably about 24 hours. The result is epoxidized thiol groups.

In a most preferred embodiment, the epoxidized keratins are cured into an elastomer using multifunctional silicone-based crosslinking agents, such as amine-functional silicones, which are available from Gelest, Inc. (Tullytown, PA). The reaction is as follows: 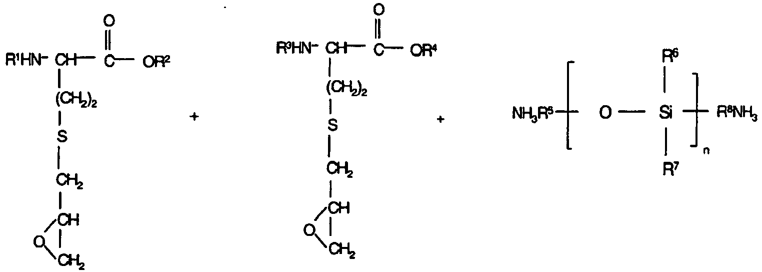 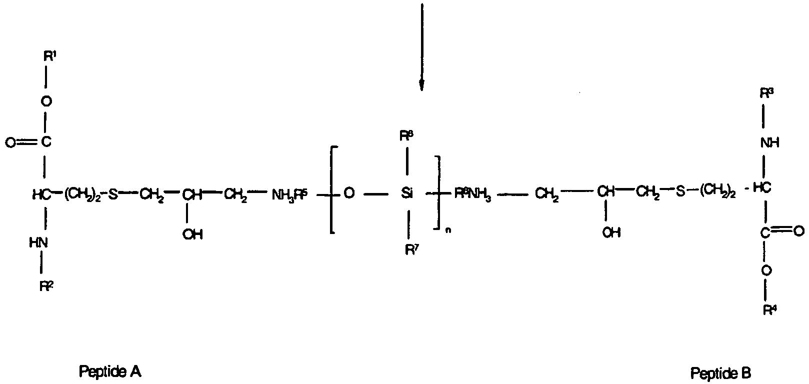 wherein R¹ and R² are the remainder of the water soluble protein "A" bearing an epoxy functionalized cysteine; R³ and R⁴ are the remainder of the water soluble protein molecule B bearing an epoxy functionalized cysteine; R⁵ and R⁸ preferably are alkyl groups having from about 1 to about 6 carbon atoms, most preferably n-propylene groups; and R⁶ and R⁷ independently are selected from the groups described above in the general formula for the silicone cross-linking agents, specifically R² and R³ thereof. Most preferably R⁶ and R⁷ are relatively inert groups, such as methyl groups. Although it is theoretically possible for water soluble protein molecule A and B to be the same molecule, it is preferred for proteins A and B to be different molecules in all of the embodiments described herein, preferably different water soluble α-keratin molecules.

In order to perform this reaction, the keratins are dissolved in water and the silicone is dissolved in a separate water-miscible organic solvent. Suitable organic solvents include ethanol, methanol, isopropyl alcohol, acetone, tetrahydrofuran, and dimethylsulfoxide. The two solutions are mixed, along with an appropriate catalyst if needed, and the mixture is cast into a film. Film drying can be accomplished by air drying, or accelerated by the application of heat or vacuum. The curing is accomplished by exposure to a source of energy, preferably heat, irradiation, or a combination thereof.

### Free Radical Addition to Reactive Pendant Groups

Addition reactions such as free radical addition to an unsaturated hydrocarbon represent another potential avenue to transformation of the thiol group. A variety of vinyl-functional silicones, for example, can be used to modify the thiol in the presence of an appropriate catalyst. Free radical catalysts can be initiated by heat or electromagnetic energy. The reaction scenario is shown below:

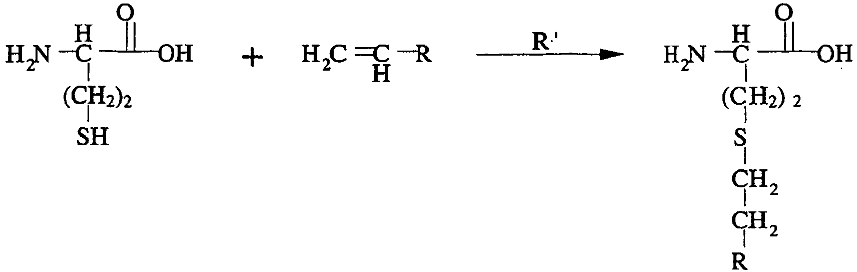

When the thiol is attached to a molecule of keratin, and if the R group of the allyl derivative is a silicone, a keratin-silicone copolymer is formed. If the silicone is at least difunctional (e.g. vinyl-terminated polydimethylsiloxane), a network or elastomeric structure results.

In order to perform this reaction, a suitable amount of keratin powder is dissolved in an anhydrous solvent, preferably comprising a suitable base. A vinyl terminated silicone fluid is added after complete dissolution, along with a suitable quantity of a free radical initiator, preferably anthraquinone-2-sulfonic acid sodium salt monohydrate. (Aldrich, Milwaukee, WI). Other suitable free radical initiators include, but are not necessarily limited to free radical photoinitiators including, but not necessarily limited to benzoin ethers, benzil ketals, α-dialkoxyacetophenones, α-hydroxyalkylphenones, α-aminoalkylphenones, acylphosphine oxides, benzophenone/amines, thioxanthones/amines, titanoocenes, and certain silanes. The amount of the vinyl functional silicone fluid added is from about 1 to about 20 weight percent relative to the amount of keratin used, preferably about 10 weight percent. The viscous solution is cast onto a suitable mold. For laboratory purposes, a suitable mold is a Teflon™ coated petri dish. The viscous solution is cured for a time effective to produce an elastomeric film having desired properties. The curing is accomplished by exposure to a source of energy, preferably heat, irradiation, or a combination thereof. In a preferred embodiment, the viscous fluid is irradiated for a period of from about 1 hour to about 4 hours, preferably about 2 hours under a UV lamp (λ = 365 nm) and then dried under a heat lamp effective to produce a temperature of at least about 60°C for a period of from about 30 minutes to about 300 minutes, preferably about 4 hours.

### Conversion of thiol by condensation

Condensation reactions such as transesterification, for example, can be used to generate thioesters of a silicone-based crosslinking agent. An example of a transesterification reaction is shown in Scheme 3. 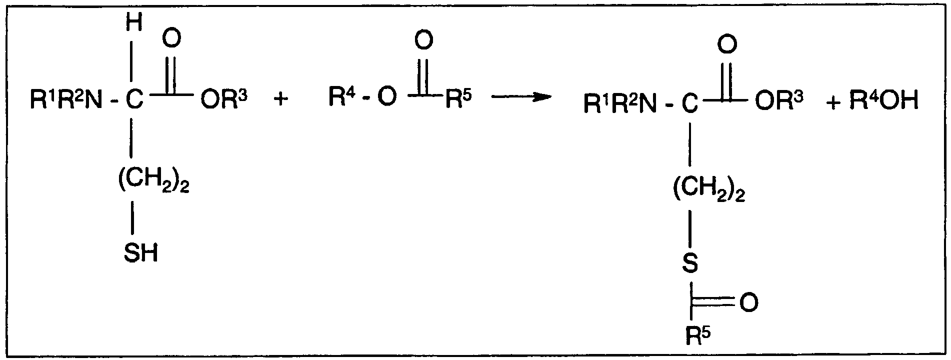 wherein R¹ and R² comprise entities selected from the group consisting of hydrogen and the remainder of the N-terminal portion of the protein molecule; R³ comprises the remainder of the carboxy-terminal portion of the protein molecule; R⁴ is an appropriate leaving group; and, R⁵ comprises a silicone-based entity. Suitable R⁴ groups include, but are not necessarily limited to hydrogen, alkyl groups having from about 1 to 6 carbon atoms, and aryl groups, including benzyl groups. R⁵ also may comprises silicone groups.

Where R⁵ is a silicone group, that group preferably has the following general structure: 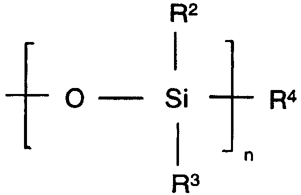 wherein n, R², R³, and R⁴ are the same as the corresponding groups described above in the general formula for the silicone cross-linking agents.

Where R⁵ comprises a silicone-based entity, the following is an exemplary reaction: 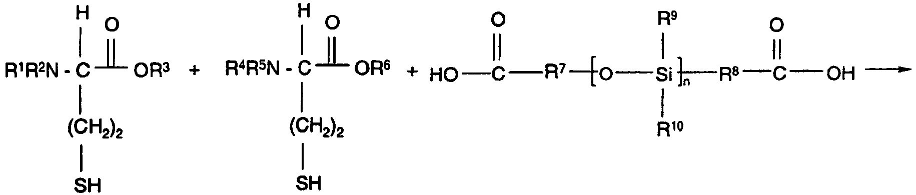 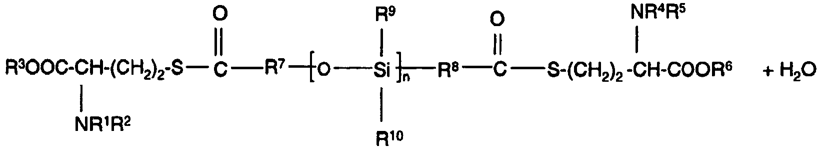 wherein n is from about 1 to about 50; R¹, R², and R³ are the remainder of a first water soluble protein molecule; R⁴, R⁵, and R⁶ are the remainder of a second water soluble protein molecule; R⁷ and R⁸ preferably are selected from the group consisting of alkyl groups having from about 1 to about 6 carbon atoms, alkoxy groups comprising one or more alkyl groups having a total of from about 1 to about 6 carbon atoms, silyl groups having the same pendant substituents as the remainder of the polymer chain (R⁹ and R¹⁰), and combinations thereof; and R⁹ and R¹⁰ independently are selected from the groups described above in the general formula for suitable silicone cross-linking agents, specifically R² and R³ thereof. Most preferably R² and R³ are relatively inert groups, such as methyl groups.

In order to perform this reaction, the keratins are dissolved in water and the silicone is dissolved in a separate water-miscible organic solvent. Suitable organic solvents include ethanol, methanol, isopropyl alcohol, acetone, tetrahydrofuran, and dimethylsulfoxide. The two solutions are mixed, along with an appropriate catalyst if needed, and the mixture is cast into a film. Film drying can be accomplished by air drying, or accelerated by the application of heat or vacuum. The curing is accomplished by exposure to a source of energy, preferably heat, irradiation, or a combination thereof.

### Addition of Amine Groups

Addition reactions between reactive amine groups and oxirane compounds occur readily without the aid of a catalyst. Preferred oxirane compounds are diepoxides having the following general structure: 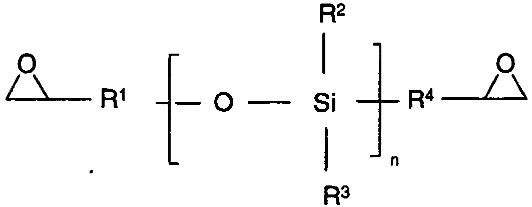 wherein n is from about 5 to about 50; R² and R³ independently are selected from the groups described above in the general formula for suitable silicone crosslinking agents, specifically R² and R³ thereof. Most preferably R² and R³ are relatively inert groups, such as methyl groups; and, R¹ and R⁴ are substantially any moieties which do not interfere with the desired characteristics of the film. Preferably, R¹ and R⁴ are selected from the group consisting of alkyl groups having from about 1 to about 3 carbon atoms, alkoxy groups comprising one or more alkyl groups having a total of from about 1 to about 6 carbon atoms, and silane groups optionally comprising one or more alkyl substituents having from about 1 to about 6 carbon atoms, most preferably methyl groups, and combinations thereof.

More preferred oxiranes have the following general structure: 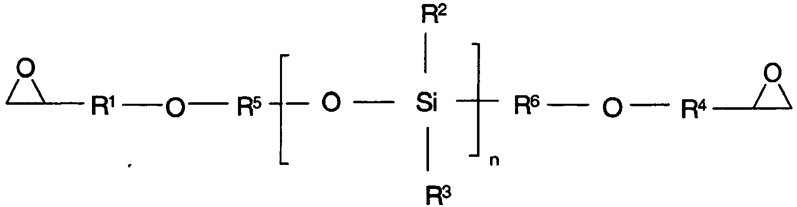 wherein n is from about 5 to about 50; R² and R³ independently are selected from the groups described above with respect to the silicone-based crosslinking agent, specifically R² and R³ thereof. Most preferably R² and R³ are relatively inert groups, such as methyl groups; and, R¹ and R⁴ are substantially any moieties which do not interfere with the desired characteristics of the film. Preferably, R¹ and R⁴ are selected from the group consisting of alkyl groups having from about 1 to about 6 carbon atoms, preferably from about 1 to about 3 carbon atoms. R⁵ and R⁶ are selected from the group consisting of alkyl groups having from about 1 to about 6 carbon atoms, preferably from about 1 to about 3 carbon atoms, silane groups optionally comprising one or more alkyl substituents having from about 1 to about 6 carbon atoms, most preferably one or more methyl substitutents, and combinations thereof.

A most preferred oxirane compound is epoxypropoxypropyl-terminated poly(dimethylsiloxane), available from Gelest, Inc., Tullytown, PA, having the structure shown below:

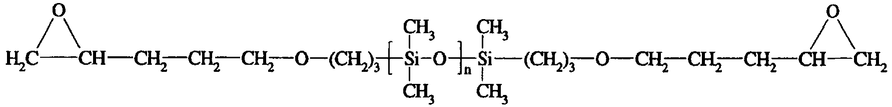

The crosslinking reaction of a diepoxy-silicone based crosslinker with arginine is as follows: 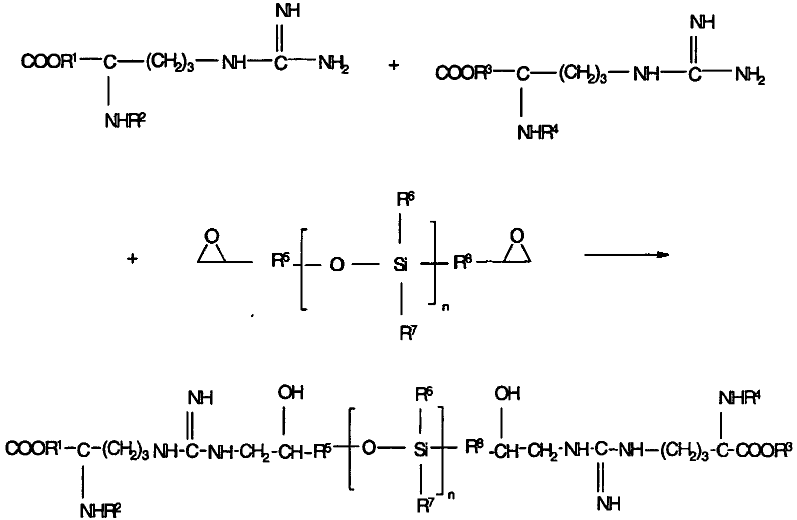 wherein R¹ and R² represent the remainder of one protein molecule; R³ and R⁴ represent the remainder of a separate protein molecule, preferably different α-keratin molecules;

In order to perform this reaction, solubilized keratins are exposed to a solution containing an oxirane-terminated silicones, such as those available from Gelest, Inc., Tullytown, PA, typically at a concentration of up to about 20 mole percent relative to keratin, preferably between 5 and 10 mole percent relative to keratin; at a pH greater than 7, preferably greater than 9, or less than 7, preferably less than 6; at a temperature of from about 0 to about 100°C, preferably about 30°C, preferably for a time period of from about 0 to about 72 hours, most preferably about 24 hours.

A similar reaction occurs when a diepoxide reacts with cysteine residues: 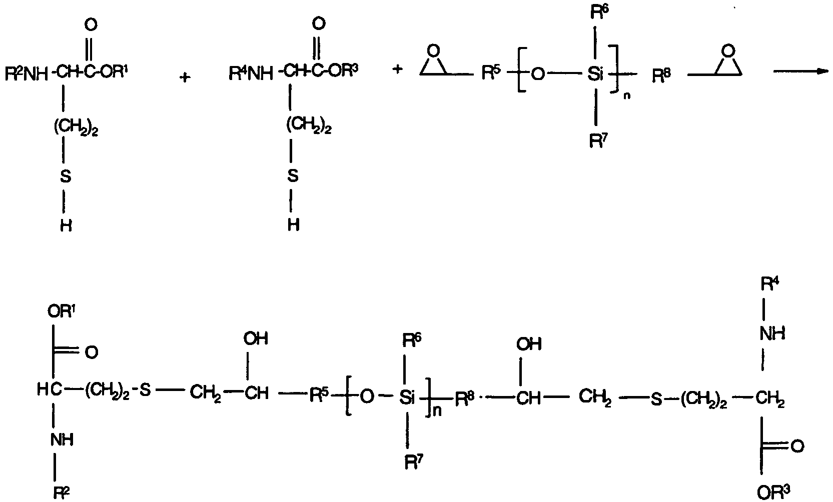 wherein R¹ and R² are the remainder of a first protein molecule and R³ and R⁴ are the remainder of a second protein molecule, preferably different α-keratin molecules. R⁵ and R⁸ are the corresponding moieties just described with respect to the reaction with arginine.

Persons of ordinary skill in the art will recognize that many of the crosslinking agents described herein will react with a variety of amino acid residues having pendant groups comprising a reactive nitrogen atom, sulfur atom, or oxygen atom. Hence, one end of a diepoxide may react with a cysteine residue while the other end of the diepoxide reacts with an arginine residue, as follows: 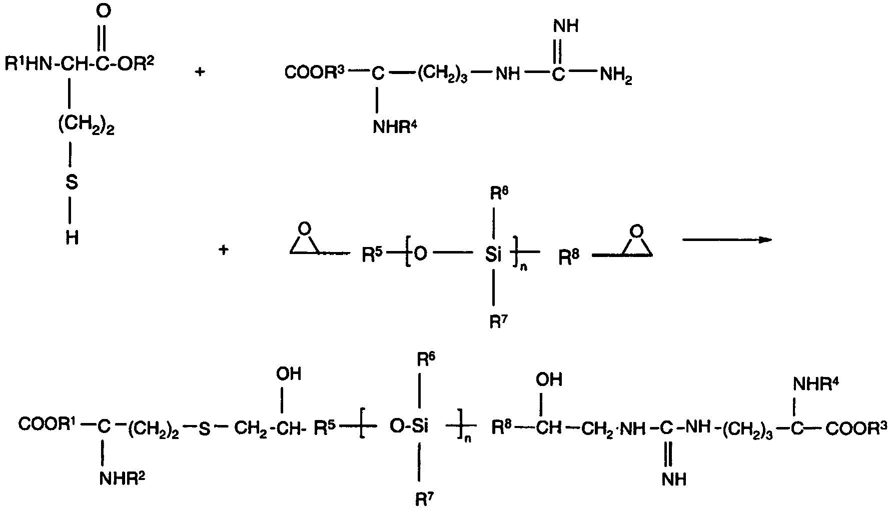 wherein R¹ and R² represent the remainder of one protein molecule; R³ and R⁴ represent the remainder of a separate protein molecule, preferably different α-keratin molecules; R⁵ and R⁸ may be any moiety which does not interfere with the desired characteristics of the film. Preferably, R⁵ and R⁸ are selected from the group consisting of propoxypropyl groups and alkyl groups having from about 1 to about 6 carbon atoms, more preferably from about 1 to about 3 carbon atoms; and R⁶ and R⁷ independently are selected from the groups described above with respect to the silicone crosslinking agents, specifically R² and R³ thereof. Most preferably R⁶ and R⁷ are relatively inert groups, such as methyl groups.

The identity of amino acid residues linked by the crosslinking agent is not as important as the requirement that a sufficient quantity of crosslinking between protein molecules occurs to produce a film having desired properties. In a preferred embodiment, the crosslinking produces an elastomeric film.

### Network properties

As seen above, a three dimensional keratin-based network can be formed using a variety of chemistries. Preferably, the "dissolution rate" of such a network is controllable by controlling the crosslink density of the film and the level and type of functionality, particularly the functionality adjacent to the crosslink site. For example, the use of a crosslinking agent having one of the following characteristics reduces the dissolution rate of the resulting network: a crosslinking agent which forms S-C bonds, as opposed to more hydrolyzable bonds, such as ester bonds; a crosslinking agent which introduces substantial steric hindrance at the crosslink site; a crosslinking agent which is hydrophobic. The "dissolution rate" of the resulting network or film is measured by determining how long the film resists hydrolysis upon exposure to an aqueous buffer having a pH of about 7. A desirable "dissolution rate" will depend upon the application in which the film is to be used.

The invention will be better understood with reference to the following Examples, which are illustrative only:

The invention is set out in the appended claims. The embodiments of the description which do not fall within the scope of the claims are provided for illustrative purposes only and do not form part of the present invention.

### Example 1

500 g of clean, dry human hair was placed in a 12 L flask with 8.35 L of 1 w/v % H₂O₂ and brought to a gentle boil. The reaction was heated without stirring at reflux for 180 minutes. The hair was filtered, rinsed with deionized water and allowed to air dry.

100 g of oxidized hair was placed in a 2 L flask with 1 L of 1M thioglycolic acid solution adjusted to pH 9 with ammonium hydroxide. The reaction was heated to 60°C under a nitrogen atmosphere with stirring for 24 hours.

The mixture of solids and liquid extract was poured into bottles under argon atmosphere. The bottles were sealed and centrifuged to affect separation of the solids. The liquid was cannulated into an 8-fold excess of cold ethanol, under nitrogen, and formed a precipitate. The precipitate was filtered, washed with ethanol, and dried under vacuum. The dried solids were ground to a powder using a mortar and pestle.

3 g of the keratin powder was dissolved into 15 mL of dimethysulfoxide (DMSO) with 1 mL of 30% ammonium hydroxide. After complete dissolution, 0.3 g of a vinyl-terminated silicone fluid (catalogue no. DMS-V03; Gelest, Inc., Tullytown, PA) and 0.1 g of anthraquinone-2-sulfonic acid sodium salt monohydrate (Aldrich, Milwaukee, WI) was added. The viscous solution was cast onto a Teflon™ coated petri dish and cured for 2 hours under a UV lamp (λ = 365 nm). The film was further dried under a heat lamp for 4 hours. This process resulted in an elastomeric film of good quality.

### Example 2

175 g of clean dry hair was placed in a 4 L glass reactor with 3.5 L of 1M mercaptoethanol adjusted to pH 10.2 with potassium hydroxide. The solution was stirred under nitrogen for 21 hours, after which, the solids were separated by filtration. The reduced hair was then extracted with 2.3 of 7M aqueous urea solution at room temperature, under nitrogen, for 24 hours.

The reaction was centrifuged and the liquid filtered, then neutralized to pH 7 by addition of concentrated hydrochloric acid. The neutralized keratin solution was added dropwise to a 10-fold excess of ethanol to affect precipitation. The precipitate was filtered, rinsed with ethanol, and dried under vacuum. The dried solid was ground to a powder using a mortar and pestle.

5 g of the ground keratin powder was dissolved in 60 g of 30% ammonium hydroxide solution with the aid of stirring, sonication, and slight heating. After complete dissolution, the ammonium hydroxide was allowed to evaporate with the assistance of a dynamic nitrogen purge. To half of this solution was added 0.05 g of anthraquinone-2-sulfonic acid sodium salt monohydrate and a solution of 0.5 g of vinyl-terminated silicone (catalogue no. DMS-V03; Gelest, Inc., Tullytown, PA) in 2 g of isopropyl alcohol. After ca. 30 minutes of stirring, the thick solution was cast onto a Teflon coated petri dish and cured under a UV lamp for ca. 3 hours. The film, was then dried under a heat lamp for ca. 1 hour. The film was placed in deionized water for 24 hours and did not dissolve.

## Claims

1. Proteinaceous film for use in wound healing, wherein said proteinaceous film is obtainable by a method comprising contacting keratin proteins of a high molecular weight of at least 50 kDa with a derivatized silicone cross-linking agent under conditions effective to form covalent bonds between reactive functional groups on the silicone cross-linking agent and thiol pendant groups of the keratin protein and drying the reaction product to produce a film, wherein the silicone crosslinking agent has the following general structure: 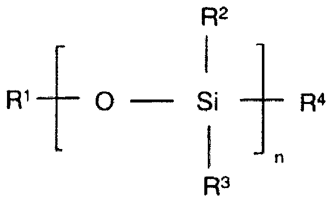 wherein n is from 1 to 50; and, at least two of R¹, R², R³, and R⁴ comprise reactive functionalities comprising vinyl groups; and wherein R¹, R², R³, and R⁴ are selected from the group consisting of hydrogen; cyclic, linear, and branched alkyl and heteroalkyl groups having from 1 to 6 carbon atoms, said groups comprising both unsubstituted groups and groups substituted with at least one reactive functionality, wherein said heteroalkyl groups comprise acetoxy groups, silane groups optionally comprising one or more alkyl substituent having a total of from 1 to 6 carbon atoms, or combinations thereof.

2. Proteinaceous film for use according to claim 1 wherein R¹ and R⁴ comprise vinyl groups.

3. Proteinaceous film for use according to claim 1-2 wherein at least one of R² and R³ is an alkyl group.

4. Proteinaceous film for use according to any of claims 1-3 wherein at least one of R² and R³ is a methyl group.

5. Proteinaceous film for use according to claim 1-4, wherein the silicone cross-linking agent is vinyl terminated polydimethylsiloxane.

6. Proteinaceous film for use according to any of claims 1-5, wherein the keratin proteins are obtained from human hair.

7. Proteinaceous film for use according to any of claims 1-5, wherein the keratin proteins are obtained from animal hair.

8. Proteinaceous film for use according to claims 6 and 7, wherein the hair is reduced.

9. Proteinaceous film for use according to claims 6 and 7, wherein the hair is oxidized.

## Patentansprüche

1. Proteinfilm zur Verwendung bei der Wundheilung, wobei der Proteinfilm erhältlich ist durch ein Verfahren umfassend das in Kontakt bringen von Keratinproteinen mit hohem Molekulargewicht von mindestens 50 kDa mit einem derivatisierten Silikon-Kreuzvernetzungsmittel unter Bedingungen, die es ermöglichen kovalente Bindungen zwischen den reaktiven funktionalen Gruppen auf dem Silikon-Kreuzvernetzungsmittel und den Thiolgruppen des Keratinproteins zu bilden, und Trocknung des Reaktionsproduktes um einen Film herzustellen, wobei das Silikon-Kreuzvernetzungsmittel die folgende allgemeine Struktur hat: 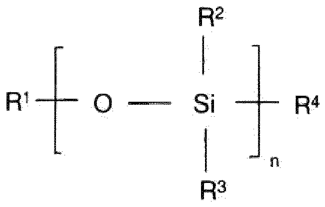 wobei n 1 bis 50 ist; und, mindestens zwei von R¹, R², R³ und R⁴ reaktive Funktionalitäten umfassend Vinylgruppen aufweisen; und wobei R¹, R², R³ und R⁴ ausgewählt sind aus der Gruppe bestehend aus Wasserstoff; zyklischen, linearen und verzweigten Alkyl- und Heteroalkylgruppen mit 1 bis 6 Kohlenstoffatomen, die Gruppen umfassen sowohl nicht substituierte Gruppen und Gruppen, die mit mindestens einer reaktiven Funktionalität substituiert sind, wobei die Heteroalkylgruppen Azetongruppen, Silangruppen optional umfassend ein oder mehrere Alkylsubstituenten mit einer Gesamtanzahl von 1 bis 6 Kohlenstoffatomen, oder Kombinationen daraus umfassen.

2. Proteinfilm zur Verwendung nach Anspruch 1, wobei R¹ und R⁴ Vinylgruppen umfassen.

3. Proteinfilm zur Verwendung nach den Ansprüchen 1-2, wobei mindestens eine der R² und R³ eine Alkylgruppe ist.

4. Proteinfilm zur Verwendung nach den Ansprüchen 1-3, wobei mindestens eine der R² und R³ eine Methylgruppe ist.

5. Proteinfilm zur Verwendung nach den Ansprüchen 1-4, wobei das Silikon-Kreuzvernetzungsmittel Vinyl-terminiertes Polydimethylsiloxan ist.

6. Proteinfilm zur Verwendung nach den Ansprüchen 1-5, wobei die Keratinproteine aus humanem Haar erhalten wurden.

7. Proteinfilm zur Verwendung nach den Ansprüchen 1-5, wobei die Keratinproteine aus tierischem Haar erhalten wurden.

8. Proteinfilm zur Verwendung nach den Ansprüchen 6 und 7, wobei das Haar reduziert ist.

9. Proteinfilm zur Verwendung nach den Ansprüchen 6 und 7, wobei das Haar oxidiert ist.

## Revendications

1. Film protéique pour utilisation dans la cicatrisation, lequel film protéique peut être obtenu par un procédé comprenant la mise en contact de protéines de type kératine de masse moléculaire élevée d'au moins 50 kDa, avec un agent de réticulation siliconé dérivé, dans des conditions efficaces pour former des liaisons covalentes entre les groupes fonctionnels réactifs sur l'agent de réticulation siliconé et les groupes thiol pendants de la protéine kératine, et le séchage du produit réactionnel pour produire un film, dans lequel l'agent de réticulation siliconé a la structure générale suivante : 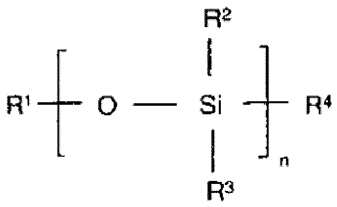 dans laquelle n vaut de 1 à 50 ; et au moins deux parmi R¹, R², R³ et R⁴ comprennent des fonctionnalités réactives comprenant des groupes vinyle ; et dans laquelle R¹, R², R³ et R⁴ sont choisis dans l'ensemble constitué par l'hydrogène ; les groupes alkyle et hétéroalkyle cycliques, linéaires et ramifiés ayant de 1 à 6 atomes de carbone, lesdits groupes comprenant tant des groupes non substitués que des groupes substitués par au moins une fonctionnalité réactive, lesdits groupes hétéroalkyle comprenant des groupes acétoxy, les groupes silane comprenant éventuellement un ou plusieurs substituants alkyle ayant au total de 1 à 6 atomes de carbone, ainsi que leurs combinaisons.

2. Film protéique pour utilisation selon la revendication 1, dans lequel R¹ et R⁴ comprennent des groupes vinyle.

3. Film protéique pour utilisation selon les revendications 1 et 2, dans lequel au moins l'un de R² et R³ est un groupe alkyle.

4. Film protéique pour utilisation selon l'une quelconque des revendications 1 à 3, dans lequel au moins l'un de R² et R³ est un groupe méthyle.

5. Film protéique pour utilisation selon les revendications 1 à 4, dans lequel l'agent de réticulation siliconé est le polydiméthylsiloxane à terminaison vinyle.

6. Film protéique pour utilisation selon l'une quelconque des revendications 1 à 5, dans lequel les protéines de type kératine sont obtenues à partir de cheveux humains.

7. Film protéique pour utilisation selon l'une quelconque des revendications 1 à 5, dans lequel les protéines de type kératine sont obtenues à partir de poils d'animaux.

8. Film protéique pour utilisation selon les revendications 6 et 7, dans lequel les cheveux ou poils sont réduits.

9. Film protéique pour utilisation selon les revendications 6 et 7, dans lequel les cheveux ou poils sont oxydés.
